# EUROPEAN PATENT SPECIFICATION

(11) **EP 2 346 387 B1**
(45) Date of publication and mention of the grant of the patent: **13.03.2013**
(21) Application number: 09785459.0
(22) Date of filing: 07.08.2009
(51) Int. Cl.: A61B 5/024, A61B 5/08, A61B 5/145

(54) **BLOOD ANALYSIS**
BLUTANALYSE
ANALYSE DE SANG

(30) Priority: 08.08.2008 GB 0814419
(43) Date of publication of application: 27.07.2011
(73) Proprietor: Health-Smart Limited, London SE1 1LB (GB)
(72) Inventor: ORBACH, Tuvi, Greater London NW11 0SA (GB); STOCKS, John Rupert, Harpenden Hertfordshire AL5 2RW (GB)
(74) Representative: Mansfield, Peter Turquand
(86) International application number: PCT/GB2009/050989
(87) International publication number: WO 2010/015863

(56) References cited:
- EP-A- 0 712 602
- JP-A- 2007 319 246

## Description

### Field of the Invention

The present invention relates to a non-invasive blood analysis method, which uses light to obtain data concerning the characteristics of an individual's blood, and to apparatus suitable for performing this method.

### Background of the Invention

It is known to have devices that can monitor the blood. Electro-optical devices for the measurement of blood characteristics have been used in many areas of blood constituent diagnosis, such as glucose levels, oxygen saturation, billirubin levels and others. A particularly well known technique for the measurement of blood characteristics is pulse oximetry. Pulse oximetry involves the transmission of two or more wavelengths of light (or infrared) through tissue at a point where blood perfuses the tissue, for example at a finger or earlobe. An LED may be the source of the light. A photodetector such as a photodiode or a photo transistor senses the absorption of light from the other side of the tissue. Often, the light sources and sensors are accommodated in a housing mounted on a clip that attaches to the body and delivers data by cables to a processor. The clips have the disadvantage that they can be uncomfortable to wear. Another type of pulse oximetry relies on reflected or back-scattered light, where a light source and a light detector are placed side by side; this can be easier to apply, as they may be placed on a part of the body without requiring a clip. There are nevertheless problems in using reflective pulse oximetry, and the requisite equipment is purpose-made and can be expensive. Ultrasound is also used to monitor flow in internal organs, but it is also requires purpose-made equipment.

For example EP 0 712 602 (Toa Medical Electronics) describes apparatus for measuring haemoglobin concentration in a patient, using a light source, and an imaging device for capturing an image of the detection region to which the light is applied. The objective lens of the imaging device is held well away from the surface of the patient's skin; and the skin is illuminated from the same side as it is viewed.

JP 2007-319246 A (Sharp) describes use of a video camera with an objective lens, such as a CCD camera in a mobile phone, for monitoring the pulses of the heart; the user may place a finger or thumb over the camera opening, and the image data is analysed to detect the changes in blood flow, and so the pulse rate.

### Statements of Invention

According to the present invention there is provided a method of performing blood analysis, using a video camera with an objective lens, the method comprising placing the video camera such that the objective lens is completely obstructed, at least partly by at least part of an appendage forming part of an individual, by placing the appendage up against a frame of the objective lens or up against a restricting frame that obstructs part of the objective lens, the method not requiring a focused image of the appendage; and operating the video camera; and characterised by analysing colour signals from the video camera to monitor colour and changes in colour and so to deduce information about blood flowing in the appendage.

Since the objective lens is completely obstructed, light can reach the objective lens only through the appendage. It will be appreciated that the method requires a source of light on the opposite side of the appendage from the video camera, but this may be ambient light, sun light or artificial light. The method requires that at least some of that light can pass through the appendage - the term appendage in this specification hence refers to a part of the human body through which some light can pass. The appendage may, for example, be an earlobe, or a fingertip, or even a fold of skin (if it is sufficiently thin that light can pass through it).

It will be appreciated that the video camera will typically be unable to produce a focused image, as the object (which in this case is the surface of the appendage) is much too close to the objective lens. Nevertheless, surprisingly, information can be obtained from the sequential camera images. In particular, from the changes in the colour information may be deduced such as changes in the oxygen or glucose level in the blood.

Preferably the camera is a portable video camera. Alternatively the camera may be a web camera. Preferably the objective lens is of diameter no more than 15 mm, more preferably no more than 10 mm, more preferably of diameter no more than about 8 mm, as it is easier to obstruct a smaller lens with a fingertip. The frame of the objective lens is an integral part of the camera; usually the lens is surrounded by a frame that projects no more than a few mm from the front surface of the lens. In some cases it may be preferable to provide a separate light-proof restricting frame, for example in the form of a short tube or ring. If the objective lens is larger than a fingertip, then a restricting frame may be provided to obstruct part of the lens, usually with a peripheral ring, to leave a smaller aperture that can be obstructed by the fingertip. Typically the restricting frame would project no more than 10 mm from the front surface of the lens, more preferably no more than 6 mm. Thus in every case the objective lens is completely obstructed; where the objective lens is sufficiently small it is completely obstructed by the appendage, whereas if the objective lens is larger it is partly obstructed by the frame and partly obstructed by the appendage. So in every case light can only reach the video camera by passing through the appendage.

In a preferred embodiment the video camera forms part of a mobile communications device such as a mobile phone, as such a mobile phone typically incorporates a video camera with an objective lens of a suitable diameter for the method of the present invention. Furthermore, software may be installed on a mobile phone to enable the signal analysis to be performed by the mobile phone itself. Hence the analysis may be carried out substantially in real-time, as the signals are being obtained.

In an alternative arrangement, signals from the mobile phone representing the video signals are transmitted to a processing unit separate from the mobile phone. This may for example be a computer. And the signals are then analysed by the processing unit.

The present invention may be used to monitor changes in the pattern of blood flow and colour in an individual over a period of time. The data received can be compared with data relating to individual conditions or diseases and a match can be made between the data received from the individual during the test and a library of data characteristics for diseases. When the comparison is made, the match between characteristics of the blood analysed for an individual and the characteristics for a particular condition or disease can be used to give an indication of a possible condition that an individual may be suffering from. Appropriate treatment can then be given to that individual and again, the blood can be analysed to see if the treatment is working. A particular advantage of the present invention is that a lay person can take the measurements with a mobile phone, without needing any medical device. The information can be analysed by software within the mobile phone and presented to the user almost in real time. Additional software can coach the user based on this information. The information can be transmitted to a medical centre, and a warning can be triggered if there are any risks. It will also save the time of healthcare professionals, and reduce the risks of transmission of infectious diseases.

### Specific Description

The invention will now be more particularly described by way of example only. In this example use is made of a mobile phone (which may be referred to in the USA as a cell phone) having a 2.0 megapixel colour video camera function (although the number of pixels may be larger or smaller than this). In one case the video camera's objective lens is of diameter about 8.0 mm and is surrounded by a frame which is non-circular, being generally square, of width 7 mm but with rounded corners, so the diagonal is of length about 8 mm, while in another case the mobile phone's objective lens is of diameter 6.0 mm, and the frame is of the same diameter. Such mobile phones are readily and widely available, and can fit in a pocket to be readily portable. In a first embodiment of the invention, image analysis software is installed on the mobile phone.

The user activates a program on the mobile phone. The program, in this example, instructs the user to place his fingertip up against the frame of the objective lens, so that the fingertip completely obstructs the objective lens, and then activate the video camera function, so that the mobile phone takes video images of the light that passes through the fingertip. It will be appreciated that this is not a focused image, because the fingertip is very close to the objective lens, but variations in both brightness and colour can be monitored. Each beat of the heart pumps blood to the periphery of the body, and slightly distends the blood flow vessels in the subcutaneous tissue. By analysing the images to observe the variation in brightness with time, each heart beat pulse can be detected, and so the pulse rate can therefore be monitored.

Blood flow to the skin can be modulated and affected by several physiological parameters. For example it is affected by breathing, as this affects the intrapleural pressure (between the thoracic wall and the lungs), leading to a variation of cardiac output during the respiration cycle. Hence the variation in the amplitude of the brightness fluctuations, with time, may be used to monitor the individual's respiration cycle.

The colour of the blood and the amount of light reflected and absorbed by specific light wavelengths (such as red or infrared) depends upon the level of oxygen saturation of the haemoglobin, and so on the concentration of oxygen, and other constituents. Oxygen-saturated haemoglobin is bright red, while unsaturated haemoglobin is darker. Analysis of the colour of the images and its changes therefore enable the oxygen saturation level to be monitored. It may also be possible to monitor and analyse changes in the level of other constituents.

This analysis is preferably carried out within the mobile phone. The resulting data, for example on pulse rate, on respiration, and on oxygen concentration, may be displayed on the mobile phone's display either numerically or graphically, or stated audibly (with a synthesised voice) e.g. "your heart rate is 65, your breathing rate is 10 per minute", or any combination of these methods. The analysis and display are preferably carried out in real time.

In a modification, which also provides for essentially real-time image analysis, the mobile phone is arranged to continuously transmit the video signals, or information based on these signals, to an external device such as a portable computer. This transmission may be wireless, or through a cable. In this case the external device carries out the analysis, and obtains the data on the physiological parameters that are being monitored. This data may be displayed by the external device, or alternatively may be transmitted back to the mobile phone to be displayed there. Indeed the data may be displayed by both the external device and by the mobile phone.

In an alternative method of operation, the mobile phone is arranged to record the video signals. The video signals are subsequently downloaded or transmitted to an external device where the analysis takes place. This does not provide real-time data. It will be appreciated that in practice the mobile phone may not only record the video signals (to enable subsequent analysis), but also transmit the video signals continuously to an external device (to enable real-time analysis). The real-time analysis within the mobile phone may be used to provide data on one physiological parameter. The subsequent analysis may be used to obtain data on other physiological parameters.

As an alternative, the signals are obtained using a dedicated portable video camera or a webcam. Such cameras are also widely and readily available, and typically provide a significantly better quality image as they have a better quality lens system with a larger optical aperture, and usually a larger number of pixels. If the objective lens is larger than a fingertip, it is necessary to provide a restricting frame around the objective lens, obstructing part of the lens, usually with a peripheral ring, to leave a smaller aperture that can be obstructed by the fingertip. As with the mobile phone, the image analysis may be carried out within the portable video camera or webcam, or alternatively the images may be transmitted or downloaded to an external device in which the analysis takes place. For example the measurements may be made with a laptop with a built-in video camera.

Where the portable video camera includes a display, rather than displaying the view seen by the camera it may display the data obtained by the signal analysis, as mentioned above. Alternatively the display may show a video, movie or film, the content of the video and its sequences being amended in accordance with changes in the heart rate of the user. For example the video might show a sportsman playing golf; if the user's heart rate indicates that they are calm and relaxed then the sportsman would be shown hitting the ball accurately, whereas if the heart rate indicates that they are worried and flustered then hitting would be inaccurate. The portable device would store in memory a multiplicity of video clips that can be assembled in different orders, and would select the next video clip in accordance with the current data about heart rate. Similarly the portable device may include an audio output, and may provide music as long as the heart rate is within acceptable limits; but if the heart rate goes outside those limits the music may be switched off or changed.

In the methods described above, the light was provided by an ambient light source. Another variation of the invention is to provide a specific source of light; this can help to achieve better diagnostics of specific conditions or blood constituents. For example the source of light can be a small torch with a specific light wavelength. It can be standard light, an LED white light, red light, infrared light, other visible and or invisible wavelength light. It is preferably a portable light source, and can be a small laser light, such as a small laser pointer. A more sophisticated version of this source of light can be a torch which can transmit more than one wavelength (e.g. has two or more sources of light, or filters that transmit different wavelengths, or an LED source producing two or more wavelengths). Such a light source may be able to switch quickly between different colours of light, or turn the light on and off very quickly.

Hence the appendage can be illuminated by a plurality of wavelengths successively and repeatedly. Preferably the light source is connected to the mobile phone (or other device) carrying out the signal analysis, for example by a USB lead or wirelessly, so the signal analysis can be synchronised with the changes in the light. In a further aspect the changes in the light may be synchronised with physiological parameters such as the heart beat; this may assist in the analysis of blood constituents.

By providing a specific source of light and analysing the changes in the spectrum of the light received by the video camera with a large number of users, while each user is also monitored by conventional medical equipment, and also storing in a database details of the medical condition of the user, his blood constituents such as oxygen saturation, CO level, CO₂ level, glucose level etc and other parameters such as perfusion index, this procedure will create a large repository of information about users, their relevant conditions and blood parameters, and the corresponding changes in the colours monitored with the video camera. By analysing this repository we can define the best sources of light with the best combination of wavelengths to diagnose specific conditions and blood constituents.

Adding such a pocket torch to a standard mobile phone with a video camera, with the appropriate software that can instruct the user and analyse the changes in brightness and colour, transforms the mobile phone into a personal medical diagnostic monitor and coach that can be used anytime anywhere. For example the mobile phone (with the video camera and software) may be used by an athlete or sportsmen to monitor his pulse during training, or may be used for relaxation monitoring and training.

In each case, as a general rule, the signals from the video camera do not represent a focused image, because the objective lens is too close to the surface of the appendage. The image data from the video camera, i.e. the signals from the image sensor, include data from which the intensities at red pixels, at green pixels, and at blue pixels can be deduced. The signal analysis may for example involve adding the intensities of all the red pixels to provide a total red intensity R; adding the intensities of all the green pixels to provide a total green intensity G; and adding the intensities of all the blue pixels to provide a total blue intensity B. Where the light source is ambient light, then these values may be scaled (by scaling factors k) and added together to provide a total intensity value T = kl.R + k2.G + k3.B. Information about blood flow can be obtained from the variation in T with time. But where the light source provides light of two different colours alternately, then the intensity values R, G and B may be analysed separately to deduce information about blood constituents.

It will be appreciated that the embodiments described above are given by way of example only and are not intended to limit the invention, the scope of which is determined by the attached claims. It is to be understood that the features described in one embodiment of the invention can be used either individually or collectively in other embodiments of the invention.

## Claims

1. A method of performing blood analysis, using a video camera with an objective lens, the method comprising:
placing the video camera such that the objective lens is completely obstructed, at least partly by at least part of an appendage forming part of an individual, by placing the appendage up against a frame of the objective lens or up against a restricting frame that obstructs part of the objective lens, the method not requiring a focused image of the appendage; and operating the video camera; and **characterised by** analysing colour signals from the video camera to monitor colour and changes in colour and so to deduce information about blood flowing in the appendage.

2. A method as claimed in claim 1 comprising illuminating the appendage with a portable light source.

3. A method as claimed in claim 2 wherein the portable light source transmits a plurality of wavelengths successively and repeatedly.

4. A method as claimed in claim 2 or claim 3 wherein the light source is connected to a device for analysing the colour signals, and the signal analysis is synchronised with the changes in light, or the changes in light are synchronised with a physiological parameter.

5. A method as claimed in any one of the preceding claims wherein the video camera is portable, forming part of a mobile communications device.

6. A method as claimed in claim 5 wherein the signal analysis is performed within the mobile communications device.

7. A method as claimed in any one of the preceding claims wherein the video camera forms part of a portable device that includes a display, and wherein data obtained by the signal analysis is displayed on the display of the device.

8. A method as claimed in any one of the preceding claims wherein the video camera forms part of a portable device that includes an audio output, and wherein data obtained by the signal analysis is output as audio information.

9. A method as claimed in any one of claims 1 to 4 wherein the video camera is a web camera.

10. A method as claimed in any one of the preceding claims wherein the information relates to at least one parameter selected from: blood flow, pulse rate, pulse shape, heart rate variability, and respiration rate.

11. A method as claimed in any one of the preceding claims wherein the information relates to blood constituents.

12. A method as claimed in any one of the preceding claims wherein the video camera is associated with a video display, and wherein the method comprises displaying to the user a video, film or movie, and amending the content of the video, film or movie or its sequences in accordance with changes in heart rate of the user as detected from the analysed signals.

13. A method as claimed in any one of the preceding claims wherein the video camera is associated with an audio output, and wherein the method comprises playing audio files of music to the user, and amending the audio output in accordance with the heart rate of the user as detected from the analysed signals.

## Patentansprüche

1. Verfahren zum Durchführen einer Blutanalyse unter Verwendung einer Videokamera mit einer Objektivlinse, wobei das Verfahren umfasst:
Platzieren der Videokamera, so dass die Objektlinse zumindest teilweise oder gänzlich durch zumindest einen Teil eines Körperglieds eines Individuums versperrt ist, indem das Körperglied so platziert wird, dass es sich gegen einen Rahmen der Objektivlinse oder einen Beschränkungsrahmen, der einen Teil der Objektivlinse versperrt, abstützt, wobei das Verfahren kein fokussiertes Bild des Körperglieds erfordert; und Betreiben der Videokamera;
und **gekennzeichnet durch** Analysieren von Farbsignalen von der Videokamera, um Farben und Farbveränderungen zu überwachen und auf diese Weise Informationen über die Durchblutung im Körperglied abzuleiten.

2. Verfahren nach Anspruch 1, das das Beleuchten des Körperglieds mit einer tragbaren Lichtquelle umfasst.

3. Verfahren nach Anspruch 2, wobei die tragbare Lichtquelle eine Vielzahl von Wellenlängen sukzessive und wiederholt überträgt.

4. Verfahren nach Anspruch 2 oder 3, wobei die Lichtquelle mit einer Vorrichtung zum Analysieren der Farbsignale verbunden ist, und wobei die Signalanalyse mit den Lichtveränderungen synchronisiert wird, oder wobei die Lichtveränderungen mit einem physiologischen Parameter synchronisiert werden.

5. Verfahren nach einem der vorstehenden Ansprüche, wobei die Videokamera tragbar und Teil eines mobilen Kommunikationsgeräts ist.

6. Verfahren nach Anspruch 5, wobei die Signalanalyse innerhalb des mobilen Kommunikationsgeräts durchgeführt wird.

7. Verfahren nach einem der vorstehenden Ansprüche, wobei die Videokamera Teil einer tragbaren Vorrichtung ist, die eine Anzeige aufweist, und wobei mithilfe der Signalanalyse erhaltene Daten auf der Anzeige der Vorrichtung angezeigt werden.

8. Verfahren nach einem der vorstehenden Ansprüche, wobei die Videokamera Teil einer tragbaren Vorrichtung ist, die eine Audioausgabe aufweist, und wobei mithilfe der Signalanalyse erhaltene Daten als Audioinformationen ausgegeben werden.

9. Verfahren nach einem der Ansprüche 1 bis 4, wobei die Videokamera eine Webkamera ist.

10. Verfahren nach einem der vorstehenden Ansprüche, wobei sich die Informationen auf zumindest einen Parameter beziehen, der aus Durchblutung, Pulsrate, Pulsform, Variabilität der Herzrate und Atmungsrate ausgewählt ist.

11. Verfahren nach einem der vorstehenden Ansprüche, wobei sich die Informationen auf Bestandteile des Blutes beziehen.

12. Verfahren nach einem der vorstehenden Ansprüche, wobei die Videokamera mit einer Videoanzeige verbunden ist, und wobei das Verfahren das Anzeigen eines Videos oder eines Films für den Benutzer und das Ändern des Inhalts des Videos oder Films oder dessen Sequenzen gemäß Änderungen der Herzrate des Benutzers, wie aus den analysierten Signalen abgeleitet, umfasst.

13. Verfahren nach einem der vorstehenden Ansprüche, wobei die Videokamera mit einer Audioausgabe verbunden ist, und wobei das Verfahren das Wiedergeben von Musikaudiodateien für den Benutzer und das Ändern der Audioausgabe gemäß der Herzrate des Benutzers, wie aus den analysierten Signalen abgeleitet, umfasst.

## Revendications

1. Procédé de réalisation d'analyse de sang, à l'aide d'une caméra vidéo ayant un objectif, le procédé comprenant :
le placement de la caméra vidéo de telle sorte que l'objectif est complètement masqué, au moins partiellement par au moins un appendice faisant partie d'un individu, par placement de l'appendice contre un corps de l'objectif ou contre un corps de restriction qui masque une partie de l'objectif, le procédé ne nécessitant pas une image focalisée de l'appendice ; et l'actionnement de la caméra vidéo ; et **caractérisé par** l'analyse de signaux de couleur provenant de la caméra vidéo pour surveiller une couleur et des changements de couleur et ainsi de déduire des informations concernant le sang circulant dans l'appendice.

2. Procédé selon la revendication 1, comprenant l'éclairage de l'appendice avec une source lumineuse portable.

3. Procédé selon la revendication 2, dans lequel la source lumineuse portable émet une pluralité de longueurs d'onde de façon successive et répétée.

4. Procédé selon l'une des revendications 2 ou 3, dans lequel la source lumineuse est reliée à un dispositif pour analyser les signaux de couleur, et l'analyse de signal est synchronisée avec les changements de lumière, ou les changements de lumière sont synchronisés avec un paramètre physiologique.

5. Procédé selon l'une quelconque des revendications précédentes, dans lequel la caméra vidéo est portable, faisant partie d'un dispositif de communication mobile.

6. Procédé selon la revendication 5, dans lequel l'analyse de signal est réalisée dans le dispositif de communication mobile.

7. Procédé selon l'une quelconque des revendications précédentes, dans lequel la caméra vidéo fait partie d'un dispositif portable qui comprend un dispositif d'affichage et les données obtenues par l'analyse de signal sont affichées sur le dispositif d'affichage du dispositif.

8. Procédé selon l'une quelconque des revendications précédentes, dans lequel la caméra vidéo fait partie d'un dispositif portable qui comprend une sortie audio et les données obtenues par l'analyse de signal sont délivrées en sortie sous forme d'informations audio.

9. Procédé selon l'une quelconque des revendications 1 à 4, dans lequel la caméra vidéo est une webcaméra.

10. Procédé selon l'une quelconque des revendications précédentes, dans lequel les informations concernent au moins un paramètre choisi parmi : la circulation sanguine, la fréquence du pouls, la forme du pouls, la variabilité de la fréquence cardiaque et la fréquence respiratoire.

11. Procédé selon l'une quelconque des revendications précédentes, dans lequel les informations concernent les constituants du sang.

12. Procédé selon l'une quelconque des revendications précédentes, dans lequel la caméra vidéo est associée à un dispositif d'affichage vidéo, et le procédé comprenant l'affichage à l'utilisateur d'une vidéo ou d'un, et la modification du contenu de la vidéo ou du film ou de ses séquences selon des changements dans la fréquence cardiaque de l'utilisateur tels que détectés à partir des signaux analysés.

13. Procédé selon l'une quelconque des revendications précédentes, dans lequel la caméra vidéo est associée à une sortie audio, et le procédé comprenant la lecture de fichiers audio de musique à l'utilisateur, et la modification de la sortie audio selon la fréquence cardiaque de l'utilisateur telle que détectée à partir des signaux analysés.
